# EUROPEAN PATENT APPLICATION

(11) **EP 4 685 480 A1**
(43) Date of publication of application: **28.01.2026**
(21) Application number: 25190581.6
(22) Date of filing: 19.07.2025
(51) Int. Cl.: G01N 33/00

(54) **GAS SENSOR ASSEMBLY AND METHOD FOR DETECTING AN OBSTRUCTION THEREOF**

(30) Priority: 22.07.2024 US 202463673918 P; 16.07.2025 US 202519270675
(71) Applicant: Therm-O-Disc, Incorporated, Westerville Ohio 43802 (US)
(72) Inventor: STARLING, Jared R., Westerville, 43802 (US)
(74) Representative: Bryn-Jacobsen, Caelia

(57) **Abstract**

A sensor assembly for detecting and/or monitoring a condition (e.g., the presence and/or accumulation of a gas) in a working environment (i.e., a sensed environment). The sensor assembly is operable to determine whether the sensor assembly is operating efficiently, inefficiently or not at all because a sensor assembly detection element is partially blocked, substantially blocked, or completely blocked from the sensed environment.

## Description

This application claims the benefit of U.S. Provisional Application No. 63/673,918, filed on July 22, 2024 and U.S. Utility Application Serial No. 19/270,675, filed on July 16, 2025.

### TECHNICAL FIELD

The present disclosure relates generally to gas sensor assemblies, systems and methods for their operation, and more particularly to a gas sensor assembly and a method for determining whether the performance of the gas sensor assembly is degraded due to an obstruction between the sensor and the working environment.

### BACKGROUND

Hydrocarbon-based refrigerants have been used as working fluids in the heat pump and refrigeration cycle of conventional air conditioning and refrigeration systems. Fluorocarbons, such as chlorofluorocarbons (CFC), hydrochlorofluorocarbons (HCFC) and hydrofluorocarbons (HFC) became commonplace in air conditioning and refrigeration systems in the 20th century due to their favorable thermodynamic properties, their non-flammability, and their non-toxicity. However, while the inert nature of many CFCs and HCFCs made them preferred choices for use as refrigerants in air conditioning and refrigeration systems for many years, that same inert nature contributed to their long lifecycles in the atmosphere. After the discovery of ozone holes in the stratosphere over the polar regions in the early 1980s, air conditioning and refrigeration systems transitioned to hydrofluorocarbon (HFC) refrigerants which were not ozone depleting, such as R-134a, R-143a, and R-410A. In the early 21st century, new refrigerants were developed to be even safer for the environment. These new refrigerants are commonly referred to as lower global warming potential (GWP) refrigerants.

The American Society of Heating, Refrigeration, and Air Conditioning Engineers (ASHRAE) has promulgated standards classifying various refrigerants according to their toxicity and flammability. For example, ASHRAE Standard 34 classifies refrigerants having a lower toxicity as Class A refrigerants, and refrigerants having a higher toxicity as Class B refrigerants. The flammability class of refrigerants is determined according to ASTM E681, Standard Test Method for Concentration Limits of Flammability of Chemicals (Vapors and Gases) at a temperature of 60° C and a pressure of 101 kPa. According to ASHRAE Standard 34, Class 1 refrigerants do not propagate a flame, Class 2L refrigerants have a lower flammability and a slow flame propagation (for example, a burning velocity less than 10 cm/s), Class 2 refrigerants have lower flammability and faster flame propagation (for example, a burning velocity of greater than 10 cm/s), while Class 3 refrigerants have a higher flammability and faster flame propagation (for example, a burning velocity greater than 10 cm/s). Under the ASHRAE Standard 34, the commonly used R-410A refrigerant has a Class A toxicity classification and a Class 1 flammability classification. Thus, R-410A is referred to as an A1 refrigerant under ASHRAE Standard 34.

New lower GWP refrigerants include, but are not limited to, refrigerants such as R-1234yf, R-1234ze, R-32, R-454A, R-454C, R-455A, R-447A, R-452B, and R-454B. These refrigerants have a Class A toxicity classification and a Class 2L flammability classification under ASHRAE Standard 34. These refrigerants may be referred to as "A2L refrigerants." Because A2L refrigerants have the ability to propagate a flame, precautions must be taken to prevent the unintentional leaking and/or accumulation of A2L refrigerant gases, particularly in enclosed spaces. However, A2L refrigerants will not ignite if their concentration level is below their lower flammability limit.

Therefore, it is desirable to provide sensor apparatus, systems, and methods for fast and reliable detection of the presence and/or accumulation of A2L refrigerants and/or other lower GWP refrigerants associated with the operation of apparatus such as air conditioning and refrigeration systems. In addition, to improve reliability and effectiveness, and ensure such apparatus or systems function according to their design, it is advantageous to be able to determine whether the normal function or performance of the sensor apparatus or system may be degraded, such as due to an obstruction between the sensor and its working environment.

### SUMMARY

New and useful systems, apparatuses, and methods for a sensor are set forth in the appended claims. Illustrative examples are also provided to enable a person skilled in the art to make and use the claimed subject matter.

The present disclosure provides a sensor assembly for detecting and/or monitoring a condition (e.g., the presence and/or accumulation of a gas) in a working environment (i.e., a sensed environment). In addition, the sensor assembly of the present disclosure is operable to determine whether the sensor assembly is operating efficiently, inefficiently or not at all because a sensor assembly detection element is partially blocked, substantially blocked, or completely blocked from the sensed environment.

The sensor assembly may include a body or housing defining an interior space of the sensor assembly (i.e., an internal environment or sensor environment). The housing may contain a detection element (e.g., a gas sensor chip), an environment change catalyst (e.g., a heater), an environmental sensor (e.g., a relative humidity and/or temperature sensor), and a controller. The detection element, environment change catalyst, and environmental sensor are each electrically connected to the controller.

The housing includes at least one opening enabling the detection element to be exposed to the sensed environment. In some configurations of the sensor assembly, the opening comprises a barrier (e.g., a semi-permeable membrane) that is configured to allow gaseous fluids to pass from the sensed environment to the sensor environment and detection element and restrict undesired particulate materials (e.g., dirt, dust, debris or other materials) from entering the sensor environment from the sensed environment.

The detection element is configured to detect and/or monitor a condition in the sensed environment and to provide sensor data or output indicative of the condition in the sensed environment. The detection element communicates with the controller to provide the sensor data.

The environment change catalyst is configured to effect or force a change in one or more ambient conditions of the sensor environment (e.g., temperature, pressure, relative humidity). The environment change catalyst communicates with the controller and its operation is controlled by the controller.

The environmental sensor is configured to detect and/or monitor a condition and/or the changes to a condition in the sensor environment. The environmental sensor communicates with the controller sensor data or an output indicative of the condition in the sensor environment and/or the changes to the condition in the sensor environment.

The controller is configured or programmed to communicate with the detection element and to request and/or receive the sensor data from the detection element. The controller is also configured or programmed to communicate with the environment change catalyst and to control the operation of the environment change catalyst. Further, the controller is configured or programmed to communicate with the environmental sensor to determine the condition and/or changes to the condition in the sensor environment. Additionally, the controller can be configured or programmed to determine whether the opening of the housing of the sensor assembly is obstructed or restricted such that the passage of gaseous fluids from the sensed environment to the sensor environment is partially, substantially or completely prohibited or blocked based on the condition in the sensor environment and/or changes to the condition of the sensor environment.

In some configurations, the sensor assembly can employ a data table (e.g., a lookup table) that can comprise empirical information. The empirical information can include values for the output of the environmental sensor that are compiled and validated by operating the sensor assembly under various but controlled conditions, including variations in the sensed and/or sensor environment (e.g., temperature, pressure, and relative humidity), variations in operation of the environment change catalyst (e.g., operational cycle times), and under simulated restriction conditions for the opening in the housing (e.g., partially blocked, substantially blocked, and completely blocked). The information in the data table can be utilized by the controller or another processor or device connected to the sensor assembly to compare with the real-time output of the environmental sensor to determine the operating condition of the sensor assembly; that is, whether the opening in the housing is obstructed or not and/or to what extent it may be obstructed.

In some configurations of the sensor assembly the detection element is an A2L refrigerant sensor. In some configurations of the sensor assembly the environment change catalyst is a resistance heater. In some configurations of the sensor assembly the environmental sensor is a relative humidity sensor. In some configurations of the sensor assembly, the controller is configured to selectively cycle power ON and OFF to the heater at predetermined time intervals (e.g., to pulse the heater) to force changes to the ambient conditions (e.g., temperature and/or relative humidity) in the sensor environment.

In some configurations of the sensor assembly, the controller is configured or programmed to receive the output from the environmental sensor, determine any change(s) to the ambient condition(s) of the sensor environment, compare the change(s) to the ambient condition(s) of the sensor environment with a data table containing values for one or more ambient conditions of the sensor environment that may be expected under specific operating conditions of the sensor assembly and, from the comparison, determine whether the opening of the housing of the sensor assembly is partially, substantially or completely obstructed and/or whether the sensor assembly is functioning normally.

In some configurations of the sensor assembly the sensor assembly is a refrigerant sensor that may include a housing, a refrigerant detection element, a heater, a relative humidity sensor, an optional temperature sensor and a main processing unit. The main processing unit comprises a printed circuit board on which is disposed a controller. The controller is configured to communicate with the refrigerant detection element, the heater, the relative humidity sensor and the optional temperature sensor and receive sensor data (e.g., output(s) from the various sensors). The housing defines an interior space (i.e., a sensor environment) and comprise an opening exposing the refrigerant detection element to the external environment (i.e., the sensed environment). The opening is operable to inhibit foreign particulate materials from passing from the external environment into the interior space of the sensor assembly and to allow gaseous fluids to pass through to the refrigerant detection element located in the interior space. The controller is configured to determine an operating condition of the sensor assembly including whether the opening is partially, substantially, and/or totally blocked. The heater is disposed within the housing and configured to heat at least a portion of the interior space of the housing under operation of the controller. The controller is configured to energize the heater, such as according to a predetermined duty cycle, for example, receive an output from the relative humidity sensor, compare that output to an expected output (e.g., a value in a data table) and determine whether or not the sensor assembly is operating normally.

In another aspect of the disclosure, a method of operating the sensor assembly includes taking a first reading of the environmental sensor to measure a first state of at least one second ambient condition in the sensor environment at a first time. Then the environment change catalyst is operated for a predetermined time period. A second reading of the environmental sensor is taken to measure a second state of the at least one ambient second condition in the sensor environment at a second time following the predetermined time period. Next, the data table is polled to find data representative of the second output of the environmental sensor. The operating condition of the sensor based on the data from the data table is then determined.

Objectives, advantages, and a preferred mode of making and using the claimed subject matter may be understood best by reference to the accompanying drawings in conjunction with the following detailed description of illustrative examples.

### DRAWINGS

The drawings described herein are for illustrative purposes only of selected examples and not all possible implementations and are not intended to limit the scope of the present disclosure.
FIG. 1 is a functional block diagram of an example of a refrigeration system used in heating, ventilation, and air conditioning systems.
FIG. 2 is a front perspective view of an example sensor assembly disposed in the refrigeration system of FIG. 1.
FIG. 3 is an exploded perspective view of the sensor assembly of FIG. 2;
FIG. 4 is a perspective view of a sensor electronics package for the sensor assembly of FIG. 2;
FIG. 5 is a perspective view of the inside of a cover of the sensor assembly of FIG. 2; and
FIG. 6 is a functional block diagram of the sensor assembly of FIG. 2.

Corresponding reference numerals indicate corresponding parts throughout the several views of the drawings, as applicable.

### DESCRIPTION

The following description of examples provides information that enables a person skilled in the art to make and use the subject matter set forth in the appended claims, but it may omit certain details already well-known in the art. The following detailed description is, therefore, to be taken as illustrative and not limiting.

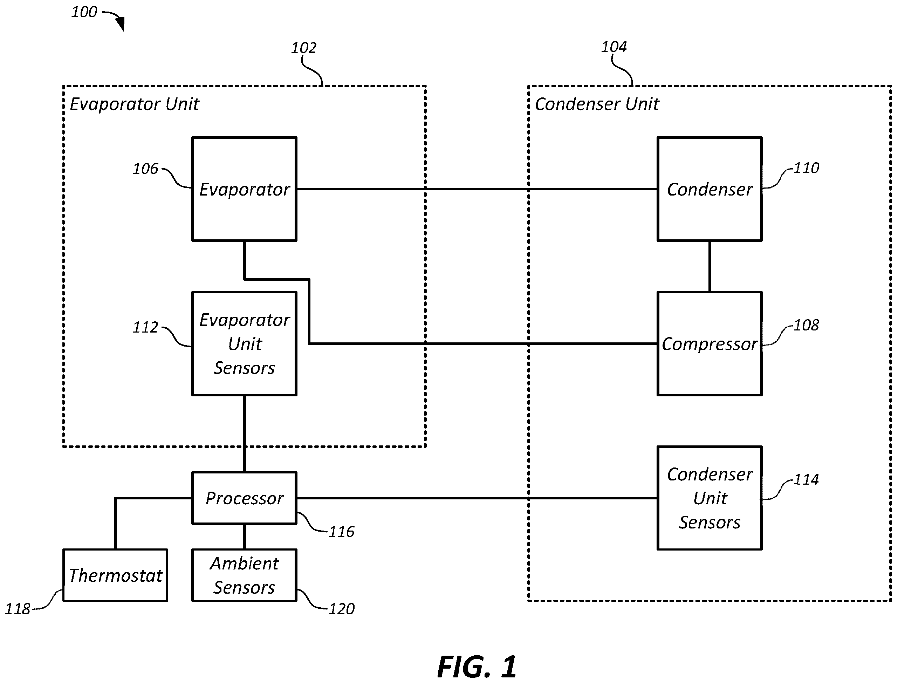

Figure 1 is a functional block diagram of an example of a refrigeration system 100 used in heating, ventilation, and air conditioning (HVAC) systems. As shown in Figure 1, some examples of the refrigeration system 100 may include a refrigeration circuit including an evaporator unit 102 and a condenser unit 104. According to some examples, the evaporator unit 102 may be located indoors and referred to as an indoor unit, while the condenser unit 104 may be located outdoors and referred to as an outdoor unit. The evaporator unit 102 may include an evaporator 106, such as an evaporator coil, and the condenser unit 104 may include a compressor 108 and a condenser 110. The evaporator 106, compressor 108, and the condenser 110 may be fluidly coupled, such as by a pipe, gas line, or liquid line. For example, the evaporator 106 may be fluidly coupled to the compressor 108 by a suction line. In some examples, the evaporator 106 may be fluidly coupled to the condenser 110 by a liquid line. According to exemplary examples, the compressor 108 may be fluidly coupled to the condenser 110 by a hot gas line.

The refrigeration system 100 may circulate a working fluid within the refrigeration circuit. The working fluid may be a lower GWP refrigerant, such as an air-to-liquid (A2L) refrigerant. For example, the A2L refrigerant may include R-1234yf, R-1234ze, R-32, R-454A, R-454C, R-455A, R-447A, R-452B, or R-454B. Alternatively, the working fluid may be another liquid such as water.

In operation, the compressor 108 may receive the working fluid through a suction port, compress the working fluid, and discharge the compressed working fluid through a discharge port. After the working fluid is compressed by the compressor 108, the working fluid gas be provided to the condenser 110 in a gas form through the hot gas line. The condenser 110 cools the working fluid, which condenses back into liquid form. The working fluid may be transported from the condenser 110 to the evaporator 106 through the liquid line. At the evaporator 106, heat is absorbed by the working fluid, causing the working fluid to expand into a gas or liquid-gas mixture. As a result of the working fluid's phase change from liquid into gas in the evaporator 106, the temperature of the working fluid is decreased, and the cooled gas may absorb heat energy from the evaporator 106, cooling the exterior of the evaporator 106 in the process. A fan (not shown) may provide airflow over the cooled exterior of the evaporator 106. As the air flows over the cooled exterior of the evaporator 106, the evaporator 106 may absorb heat energy from the flowing air, cooling the air. This cooled air may then be provided via ductwork to an air-conditioned environment, such as the interior of a room within a building.

The refrigeration system 100 may also include various monitoring and control means, such as sensors, thermostats, and processors. For example, evaporator unit sensors 112 may be provided within a housing member of the evaporator unit 102, and condenser unit sensors 114 may be provided within a housing member of the condenser unit 104. The evaporator unit sensors 112 and condenser unit sensors 114 may be operatively coupled to a controller 116 (e.g., a processor). In some examples, a thermostat 118 may be provided to monitor the air-conditioned environment. The thermostat 118 may also be operatively coupled to the controller 116. In illustrative examples, additional ambient sensors 120 may also be provided and operatively coupled to the controller 116.

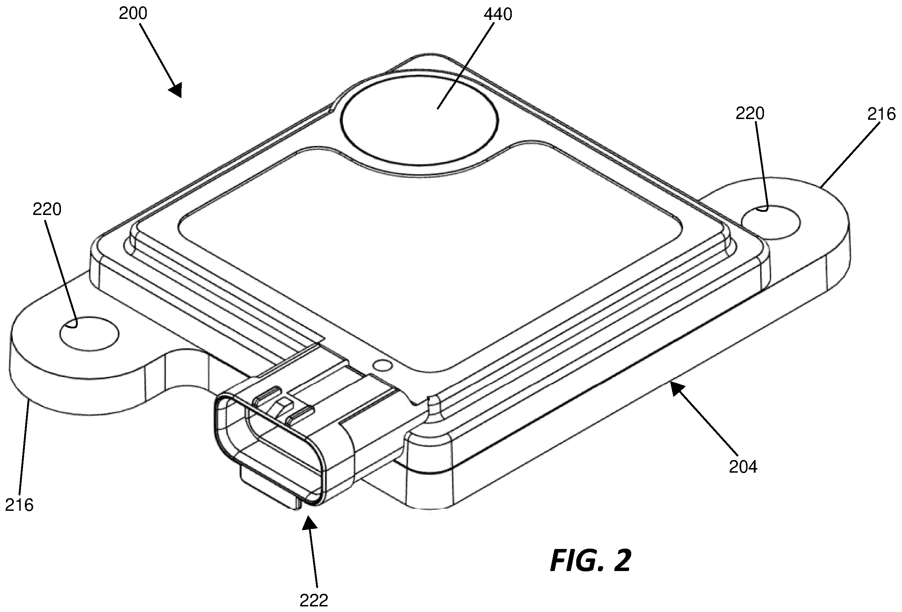

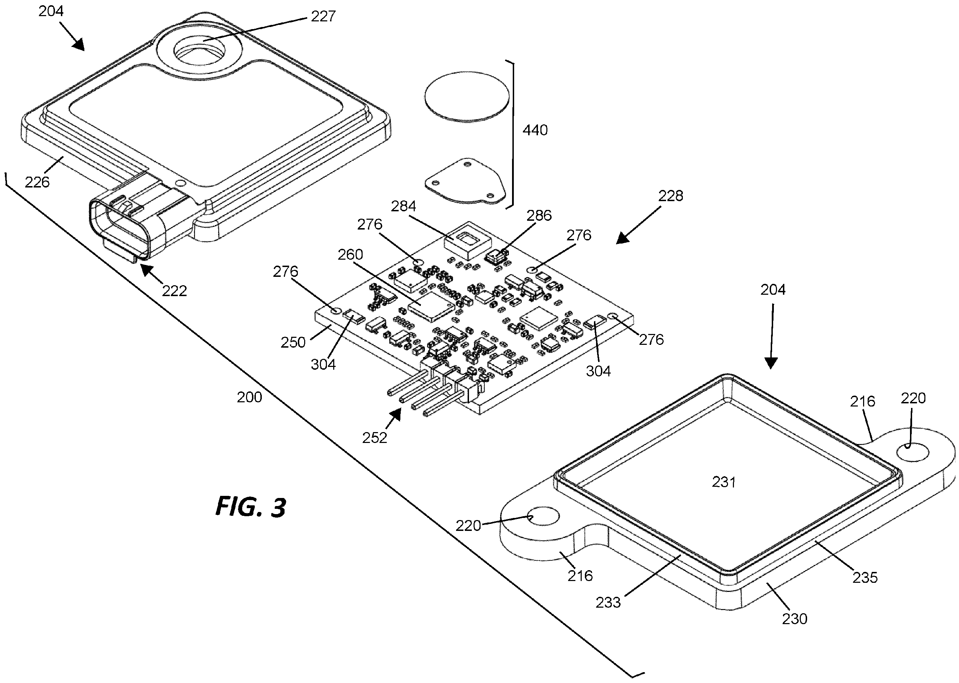

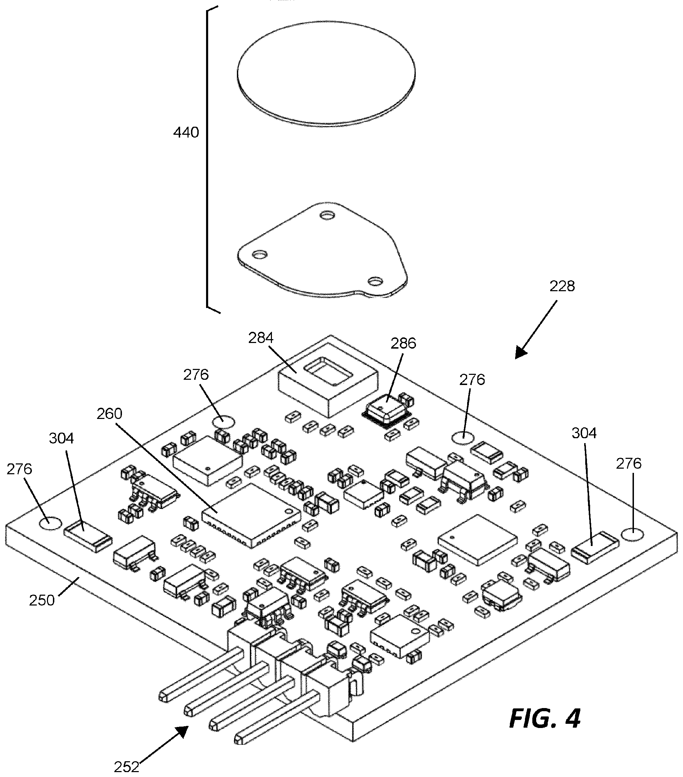

Now referring to Figures 2 to 4, an exemplary sensor assembly 200 is illustrated. The sensor assembly 200 may be a sensor of the evaporator unit sensors 112, a sensor of the condenser unit sensors 114, or a thermostat 118, as previously described. The sensor assembly 200 may be a gas sensor, such as an A2L refrigerant sensor or other gas sensor. For example, the sensor assembly 200 may be disposed in and/or exposed to a working environment to be sensed (i.e., the sensed environment) such as in or near the evaporator coil and may detect the presence and/or accumulation of the working fluid (for example, in a gaseous state), and particularly the A2L refrigerant, in the sensed environment (i.e., and outside of the refrigeration circuit) which is indicative of a leak in the refrigeration circuit and/or other failure of the refrigeration system.

The sensor assembly 200 can provide a housing 204 encasing a sensor electronics package 228 within an interior space of the sensor assembly (i.e., a sensor environment). The sensor electronics package 228 can include a detection element (e.g., a gas sensor 284), an environment change catalyst (e.g., one or more heaters 304), an environmental sensor (e.g., a relative humidity sensor 286 and/or temperature sensor 308), and a programmed or programmable controller 260, all disposed on a printed circuit board (PCB) 250.

The housing 204 can include an opening 227 allowing the sensor environment to freely communicate with the sensed environment. The opening also exposes the detection element 284 to the sensed environment and enables the detection element 284 to sense an undesired condition(s) in the sensed environment, such as the presence or accumulation of a working fluid.

The environment change catalyst (e.g., including heater(s) 304) can also be operated by the controller 260 to obtain and/or maintain a temperature in the sensor environment above a dewpoint of the sensed environment, and the environmental sensor can provide outputs or data enabling the controller 260 or another processor associated with the sensor assembly 200 to compensate an output from the sensor assembly 200 for the ambient conditions (e.g., temperature, humidity, and/or pressure) in the sensed environment.

In addition, in the sensor assembly 200 of the present disclosure the controller 260 can employ the environment change catalyst 304 to force known changes in the ambient conditions of the sensor environment (e.g., temperature and/or relative humidity) by controlling operation of the environment change catalyst 260 according to predetermined or programmed duty cycles (e.g., pulsing the heater(s) ON/OFF), and simultaneously monitoring output of the environmental sensor (e.g., temperature and/or relative humidity). As such, the sensor assembly 200 of the present disclosure can evaluate the changes to the ambient conditions (e.g., relative humidity) of the sensor environment over time.

If the sensor assembly 200 and/or the detection element 284 is or becomes restricted (e.g., partially, substantially or completely blocked) from the sensed environment, such as under conditions where the opening 227 in the housing 204 accumulates a build-up of foreign materials (e.g., particulates like dirt, dust, and lint, and liquids like oil, condensation, etc.), the sensor environment cannot freely communicate with the sensed environment. Applicant has appreciated that one consequence of such a restriction is that water vapor present in the confined space (or volume) of the sensor environment (e.g., as measured by the relative humidity) cannot readily be exchanged with or dispersed into the comparatively very large space (or volume) of the sensed environment through the opening 227, and, therefore, that the relative humidity in the sensor environment will not change in response to the change in temperature of the sensor environment as would be anticipated or expected if the sensor environment were able to freely communicate with the sensed environment.

Applicant has, therefore, determined that by utilizing the environmental sensor (e.g., a relative humidity sensor 286 and/or temperature sensor 308) to monitor one or more ambient conditions in the sensor environment (e.g., the relative humidity and/or temperature) while employing the environment change catalyst (e.g., a heater) to force a corresponding a known or predicted change to the sensor environment (e.g., increasing the temperature by turning ON the heater) and then comparing a subsequent output from the environmental sensor (e.g., representing a change in the relative humidity) with an expected output from the environmental sensor (e.g., representing or reflective of an expected or predicted change in relative humidity due to the temperature increase), the sensor assembly 200 of the present disclosure can be configured to determine whether the sensor assembly 200 is operating efficiently, inefficiently or not at all because the opening 227 in the housing 204 is open, partially blocked, substantially blocked, or completely blocked.

An expected or predicted output of the environmental sensor (e.g., representing or reflective of a change in relative humidity of the sensor environment) can comprise information (e.g., empirical data) from a data table (e.g., a look-up table) compiled and validated by operating the sensor assembly under various but controlled conditions, including variations in the ambient environment (e.g., temperature, pressure, and relative humidity), heater cycling (i.e., power ON and power OFF time intervals), and under simulated restriction conditions of the opening 227 in the housing 204 (e.g., a partial, substantial or complete blockage of the opening 227), and recording or storing the output of the environmental sensor (e.g., a relative humidity sensor 286 and/or temperature sensor 308). The data table can be utilized (e.g., searched or polled) by the controller 260 or another processor or device connected to the sensor assembly 200 to compare with the subsequent output from the environmental sensor, and from that comparison determine the operating condition of the sensor assembly 200.

The housing 204 of the sensor assembly 200 can define the interior space of the sensor assembly (i.e., the sensor environment). The housing 204 can include one or more exterior tabs 216. The tabs 216 can project laterally outwardly and include apertures 220 for mounting or fixing the housing 204 to a support structure in the working environment (e.g., a refrigeration system 100).

The housing 204 can have a multi-piece, clamshell construction including a cover 226 and a base 230. The base 230 may be formed integrally, and monolithically with the tabs 216. The base 230 may define a recess or cavity 231 for receiving the electronics package 228.

The cover 226 may close and seal the recess or cavity 231 in the base 230 to enclose the interior space of the sensor assembly (i.e., the sensor environment) from the outside, working environment (i.e., the sensed environment). The cover 226 may include a skirt portion 229 that is configured to fit over a stepped portion or rib 233 of the base 230 such that the cover 226 can seal against the base 230. The cover 226 can seal against the base 230 at a surface 235. If desired, a seal such as a gasket or O-ring can be disposed between the cover 226 and the base 230 to further effect a seal.

The base 230 and cover 226 each can be formed from plastic, metal, or any other suitable material or combination of materials. The base 230 and cover 226 may each be formed by injection molding or in another suitable manner.

The structure of the housing 204 may allow the sensor assembly 200 to be positioned in a harsh environment, for example the evaporator 102 of a refrigeration system 100. The housing 204 may be solid for protecting the sensor electronics package 228. The housing 204 may also provide a port 222 in a side of the cover 226 to provide access to a connector 252 of the electronics package 228.

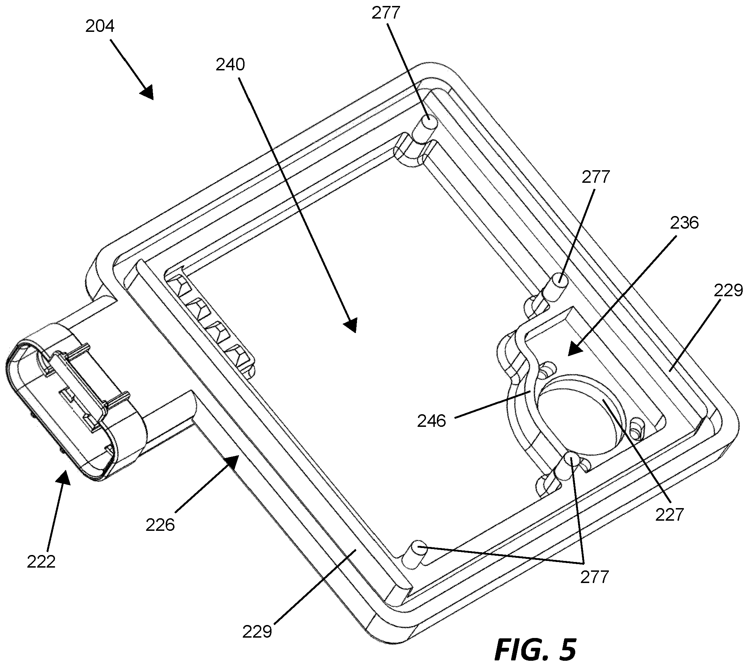

As best seen in FIG. 5, the housing 204 may optionally include one or more barriers 246. The barrier(s) 246 may separate the interior space of the sensor assembly 200 into a first cavity 236 containing the detection element (e.g., gas sensor 284) and the environmental sensor (e.g., the RH sensor 286 and/or temperature sensor 308), and a second cavity 240 containing the remainder of the electronics package 228, including the environment change catalyst (e.g., heater(s) 304). Alternatively, the environment change catalyst (e.g., heater(s) 304) can be contained in the first cavity 236 and disposed near the detection element. The barrier(s) 246 can be projection(s) integral with and extending from an underside of the cover 226 of the housing 204. Depending upon the location of the sensors, the barrier(s) 246 may provide sealing between the first cavity 236 and the second cavity 240.

The housing 204, and particularly the cover 226 of the housing 204, may include an opening 227 into the first cavity 236 to allow at least the detection element to be exposed to the sensed environment. One or more membrane(s) and/or filter(s) 440, can be positioned over and cover the opening 227. The membrane(s) and/or filter(s) 440 can be semi-permeable barriers and allow gas and/or vapor to pass from the sensed environment through the opening 227 and into the cavity 236 to the detection element, but can also serve to minimize or prohibit foreign materials, including particulates like dirt, dust, and lint, and liquids like oil, condensation, etc. that may be present in the sensed environment, from entering into the first cavity 236 and negatively affecting the operation of the detection element. The membrane(s) and/or filter(s) 440 may be formed of an elastomer, fabric, or other suitable material.

In addition to the detection element (e.g., a gas sensor 284), the environment change catalyst (e.g., one or more heaters 304), the environmental sensor (e.g., a relative humidity sensor 286 and/or temperature sensor 308), and the controller 260, the sensor electronics package 228 can include other well-known and understood electronic components, such as a power supply, switches, filter/signal conditioner(s), A/D converter(s), LED(s), and a connector 252, mounted on the printed circuit board 250. The printed circuit board 250 may be sized to fit within the cavity 231 and be captured within the interior space of the housing 204. The first printed circuit board 250 may include one or more apertures 276 with which corresponding posts 277 of the cover 226 and/or base 230 can engage for mounting within the housing 204.

The connector 252 may be a universal serial bus (USB) to serial (such as transistor-transistor logic, TTL interface) converter (for example, a Future Technology Devices International^{™}, FTDI^{™}) connector configured to receive a cable and transmit a sensor output or data from the sensor assembly 200. The connector 252 may be positioned on an end of the first printed circuit board 250 such that the connector 252 aligns with the opening 222 in the cover 226 of the housing 204.

The controller 260 may communicate with the detection element, the environment change catalyst, the environmental sensor, and the other components of the sensor electronics package 228 to control the various functions and operations of the sensor assembly 200. The controller 260 may transmit a sensor output or data from the sensor assembly 200 through the connector 252. A wiring harness connected to the connector 252 may transmit the sensor output from the connector 252 to various external controllers or devices.

In addition to a role in the function of determining whether the sensor assembly 200 is functioning efficiently, the heater 304 can also be used to mitigate condensing moisture within the interior space of the sensor assembly 200, and particularly including the cavity 236. Typical condensing of relative humidity (RH) may occur in colder temperatures, for example ambient air temperatures less than 25°C. During the summer, water may build in the evaporator coil because the coil temperature is less than a dew point temperature of the inlet air. When a sensor, such as sensor assembly 200 is attached to the evaporator coil assembly, water may build within the cavities 236, 240 for at least the same reasons. The heater 304 may mitigate the condensing moisture by internally heating the sensor electronics to keep the sensor temperature from dropping below the dew point temperature.

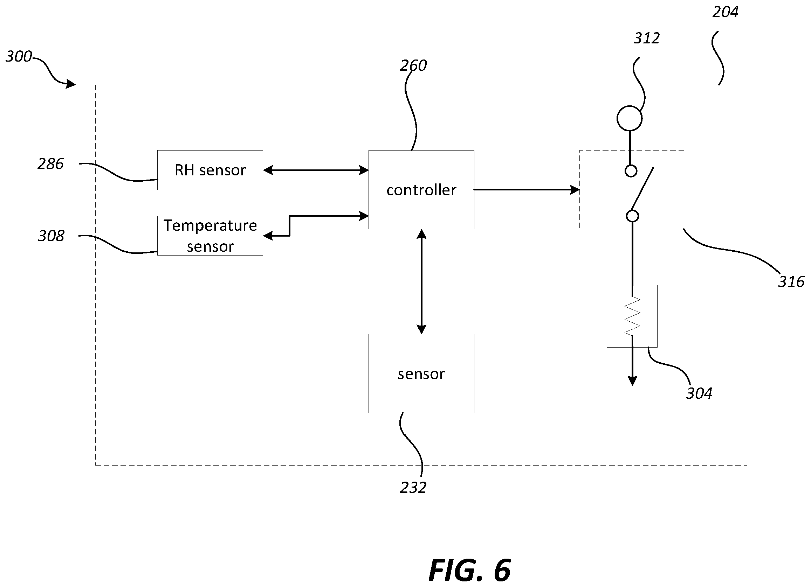

Now referring to FIG. 6, a functional block diagram of the sensor assembly 200 is illustrated. The sensor assembly 200 includes the housing 204 that encloses the controller 260, the RH sensor 286, the gas sensor 284, a temperature sensor 308, a power source 312, a relay or switch 316, and the heater 304.

The controller 260 may be in communication with the switch 316 and may be configured to energize the switch 316 to connect the power source 312 to the heater 304. The controller 260 may actuate and de-actuate the switch 316 to pulse power from the power source 312 to the heater 304 according to a predetermine or preferred duty cycle.

The controller 260 may be configured to request and receive sensor data from the RH sensor 286, the temperature sensor 308 and/or the gas sensor 284. More specifically, the sensor data may include outputs or readings from one or both of the RH sensor 286 and the temperature sensor 308. The sensor data may further include an output from the gas sensor 284 (e.g., an A2L sensor chip).

The controller 260 may process the sensor data to transmit the sensor output through the connector 252. Additionally, the controller 260 (or an external device in communication with the controller), using the sensor data, may determine whether the sensor assembly 200 is functioning effectively, including whether communication between the detection element and the sensed environment is partially or completely blocked.

A restriction or blockage condition can be determined by the controller 260 (or another, external controller or device connected to the sensor assembly 200 via the connector 252), when the change in relative humidity 286 of the sensor environment brought about by the controlled operation of the heater 304 is not consistent an expected or predicted change in relative humidity in the sensor environment due to the change in temperature of the sensor environment.

Therefore, to determine whether the sensor assembly 200 is operating efficiently, inefficiently or not at all because the opening 227 in the housing 204 is open, partially blocked, substantially blocked or completely blocked can include the following.

Taking a first reading of the environmental sensor to measure a first state of the at least one second ambient condition in the sensor environment at a first time. Then the environment change catalyst is operated for a predetermined time period. Subsequently, a second reading of the environmental sensor is taken to measure a second state of the at least one ambient second condition in the sensor environment at a second time following the predetermined time period. Then, the data table is polled for data representative or reflective of, or correlated to, the second output of the environmental sensor. Thereafter, the operating condition of the sensor then can be determined utilizing the data table.

The first reading of the environmental sensor can be the relative humidity in the sensor environment. The environment change catalyst can be a heater that is cycled to force a known change to the sensor environment (e.g., cycling the heater ON for a predetermined time period to affect an increase in the ambient temperature of the sensor environment to correspondingly affect the relative humidity of the sensor environment). The second reading of the environmental sensor (e.g., at the end of the predetermined time period) can measure the relative humidity again. The controller can then poll (or search) the data table for data representative or reflective of, or correlated to, the readings of the environmental sensor.

The output of the environmental sensor (e.g., a change in relative humidity) can be correlated to empirical information stored in a data table. The empirical information can be compiled and validated by operating the sensor assembly under various but controlled conditions, including variations in the ambient environment (e.g., temperature, pressure, and relative humidity), heater cycling (i.e., power ON and power OFF time intervals), and under simulated restriction conditions for the opening 227 in the housing 204, and recording the output of the environmental sensor.

The data table can then be utilized by the controller 260 or another processor or device connected to the sensor assembly 200 to compare with the real-time output from the environmental sensor to determine the operating condition of the sensor assembly 200; that is, whether the opening 227 in the housing 204 is obstructed or not. In this respect, for example, the data table can be polled (e.g., searched) based on the real-time output from the environmental sensor to determine the corresponding condition of the opening in the housing that is correlated to the environmental sensor output.

In addition to, or alternatively, the controller 260 can actuate the heater 304 to mitigate condensation in the sensor assembly 200 as described in Applicant's U.S. Patent No. 12,241,810 entitled, "Sensor Assembly.". Moreover, the method for determining an obstruction in the sensor assembly described herein can be applied to other sensor constructions, including sensors as described in Applicant's aforementioned U.S. Patent No. 12,241,810 and in Applicant's U.S. Patent No. 12,275,575 entitled, "Sensor Assembly and Refrigerant Sensing System."

Further examples are set out in the clauses below:
CLAUSE 1: A sensor for detecting a condition in a sensed environment comprising: a housing defining an interior space of the sensor comprising a sensor environment, a detection element configured to detect at least one first ambient condition in the sensed environment and provide a first output indicative of the at least one first ambient condition in the sensed environment; an environment change catalyst; an environmental sensor configured to detect at least one second ambient condition in the sensor environment and provide a second output indicative of the at least one second ambient condition in the sensor environment; a controller configured to communicate with the detection element, the environment change catalyst, and the environmental sensor; and wherein the detection element, environment change catalyst, and environmental sensor are located within the housing; wherein the housing includes an opening exposing the detection element to the sensed environment comprising a barrier that is configured to allow gaseous fluids to pass from the sensed environment to the detection element; a data table comprising data obtained by operating the sensor under a plurality of controlled conditions; wherein the controller is configured to determine an operating condition of the sensor.
CLAUSE 2: The sensor of clause 1 wherein the controller is configured to poll the data table based on the second output of the environmental sensor to determine the operating condition of the sensor.
CLAUSE 3: The sensor of clause 2 wherein the data in the data table comprises empirical data correlated to the second output of the environmental sensor.
CLAUSE 4: The sensor of clause 3 wherein the data in the data table comprises empirical data correlated to the second output of the environmental sensor obtained by operating the sensor under a plurality of controlled ambient conditions in the sensor environment and simulated restriction conditions for the opening in the housing.
CLAUSE 5: The sensor of clause 4 wherein the controller is configured to determine whether the opening is restricted such that passage of gaseous fluids from the sensed environment through the opening is at least one of partially prohibited, substantially prohibited or completely prohibited.
CLAUSE 6: The sensor of any one of the preceding clauses wherein the controller is configured to compare the second output of the environmental sensor with the data in the data table to determine whether the opening in the housing is obstructed.
CLAUSE 7: The sensor of clause 6 wherein the data in the data table comprises empirical data representing the second output of the environmental sensor obtained by operating the sensor under a plurality of controlled ambient conditions in the sensor environment and simulated restriction conditions for the opening in the housing.
CLAUSE 8: The sensor of any one of the preceding clauses wherein the controller is configured to control operation of the environment change catalyst to effect a change in the at least one second ambient condition in the sensor environment; wherein the environmental sensor is configured to provide the second output at a first time and at a second time; and wherein the controller is configured to receive the second output of the environmental sensor at the first time and the second output of the environmental sensor at the second time.
CLAUSE 9: The sensor of clause 8 wherein the controller is configured to compare at least one of the second output of the environmental sensor provided at the first time and the second output of the environmental sensor provided at the second time with the data in the data table to determine whether the opening in the housing is obstructed.
CLAUSE 10: The sensor of clause 9 wherein the data in the data table comprises empirical data reflective of the second output of the environmental sensor obtained by operating the sensor under a plurality of controlled ambient conditions in the sensor environment and simulated restriction conditions for the opening in the housing.
CLAUSE 11: The sensor of clause 10 wherein the controller is configured to determine whether the opening in the housing is restricted such that passage of gaseous fluids from the sensed environment through the opening is at least one of partially prohibited, substantially prohibited or completely prohibited.
CLAUSE 12: The sensor of clause 11 wherein the detection element is an A2L refrigerant sensor; wherein the environment change catalyst is a resistance heater; wherein the environmental sensor is a humidity sensor; and wherein the controller is configured to cycle power to the resistance heater to effect a change in the at least one second ambient condition of the sensor environment.
CLAUSE 13: The sensor of clause 12 wherein the opening comprises a semi-permeable barrier.
CLAUSE 14: The sensor of any one of clauses 8-13 wherein the controller is configured to determine a change in the at least one second ambient condition in the sensor environment, compare the change in the at least one second ambient condition in the sensor environment with the data in the data table, and determine the operating condition of the sensor including at least one of whether the opening of the housing of the sensor is restricted and whether the sensor is functioning normally.
CLAUSE 15: The sensor of clause 14 wherein the controller is configured to determine whether the opening of the housing is restricted such that passage of gaseous fluids from the sensed environment through the opening is at least one of partially prohibited, substantially prohibited, or completely prohibited.
CLAUSE 16: A method for operating the sensor of any one of clauses 1-15 comprising: taking a first reading of the environmental sensor to measure a first state of the at least one second ambient condition in the sensor environment; operating the environment change catalyst; taking a second reading of the environmental sensor to measure a second state of the at least one ambient second condition in the sensor environment; polling the data table to find data representative of the second output of the environmental sensor; and determining the operating condition of the sensor based on the data from the data table.
CLAUSE 17: The method of clause 16 wherein determining the operating condition of the sensor comprises determining at least one of whether the opening of the housing of the sensor is restricted and whether the sensor is functioning normally.
CLAUSE 18: The method of clause 16 wherein determining the operating condition of the sensor comprises determining whether the opening in the housing is restricted such that passage of gaseous fluids from the sensed environment through the opening is at least one of partially prohibited, substantially prohibited, or completely prohibited.
CLAUSE 19: The method of any one of clauses 16-18 further comprising comparing the first state to the second state and determining a change in state of the at least one second ambient condition of the sensor environment; and wherein polling the data table comprises finding data representative of the change in state of the at least one second ambient condition of the sensor environment.
CLAUSE 20: A method for operating the sensor of any one of clauses 12-19 comprising: taking a first reading of the environmental sensor to measure a first state of the at least one second ambient condition in the sensor environment at a first time; cycling power to the environment change catalyst; taking a second reading of the environmental sensor to measure a second state of the at least one ambient second condition in the sensor environment at a second time; polling the data table to find data representative of the second output of the environmental sensor; and determining the operating condition of the sensor based on the data from the data table.

While shown in these illustrative examples, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use.
The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art. Features, elements, and aspects described in the context of some examples may also be omitted, combined, or replaced by alternative features serving the same, equivalent, or similar purpose.

## Claims

1. A sensor for detecting a condition in a sensed environment comprising:
a housing defining an interior space of the sensor comprising a sensor environment,
a detection element configured to detect at least one first ambient condition in the sensed environment and provide a first output indicative of the at least one first ambient condition in the sensed environment;
an environment change catalyst;
an environmental sensor configured to detect at least one second ambient condition in the sensor environment and provide a second output indicative of the at least one second ambient condition in the sensor environment;
a controller configured to communicate with the detection element, the environment change catalyst, and the environmental sensor; and
wherein the detection element, environment change catalyst, and environmental sensor are located within the housing;
wherein the housing includes an opening exposing the detection element to the sensed environment comprising a barrier that is configured to allow gaseous fluids to pass from the sensed environment to the detection element;
a data table comprising data obtained by operating the sensor under a plurality of controlled conditions;
wherein the controller is configured to determine an operating condition of the sensor.

2. The sensor of claim 1 wherein the controller is configured to poll the data table based on the second output of the environmental sensor to determine the operating condition of the sensor.

3. The sensor of claim 2 wherein the data in the data table comprises empirical data correlated to the second output of the environmental sensor.

4. The sensor of claim 3 wherein the data in the data table comprises empirical data correlated to the second output of the environmental sensor obtained by operating the sensor under a plurality of controlled ambient conditions in the sensor environment and simulated restriction conditions for the opening in the housing.

5. The sensor of claim 4 wherein the controller is configured to determine whether the opening is restricted such that passage of gaseous fluids from the sensed environment through the opening is at least one of partially prohibited, substantially prohibited or completely prohibited.

6. The sensor of any one of the preceding claims wherein the controller is configured to compare the second output of the environmental sensor with the data in the data table to determine whether the opening in the housing is obstructed; and
wherein the data in the data table comprises empirical data representing the second output of the environmental sensor obtained by operating the sensor under a plurality of controlled ambient conditions in the sensor environment and simulated restriction conditions for the opening in the housing.

7. The sensor of any one of the preceding claims wherein the controller is configured to control operation of the environment change catalyst to effect a change in the at least one second ambient condition in the sensor environment;
wherein the environmental sensor is configured to provide the second output at a first time and at a second time;
wherein the controller is configured to receive the second output of the environmental sensor at the first time and the second output of the environmental sensor at the second time;
wherein the controller is configured to compare at least one of the second output of the environmental sensor provided at the first time and the second output of the environmental sensor provided at the second time with the data in the data table to determine whether the opening in the housing is obstructed; and
wherein the data in the data table comprises empirical data reflective of the second output of the environmental sensor obtained by operating the sensor under a plurality of controlled ambient conditions in the sensor environment and simulated restriction conditions for the opening in the housing.

8. The sensor of claim 7 wherein the controller is configured to determine whether the opening in the housing is restricted such that passage of gaseous fluids from the sensed environment through the opening is at least one of partially prohibited, substantially prohibited or completely prohibited.

9. The sensor of claim 8 wherein the detection element is an A2L refrigerant sensor;
wherein the environment change catalyst is a resistance heater;
wherein the environmental sensor is a humidity sensor; and
wherein the controller is configured to cycle power to the resistance heater to effect a change in the at least one second ambient condition of the sensor environment; and
wherein the opening comprises a semi-permeable barrier.

10. The sensor of claim 9 wherein the controller is configured to determine a change in the at least one second ambient condition in the sensor environment, compare the change in the at least one second ambient condition in the sensor environment with the data in the data table, and determine the operating condition of the sensor including at least one of whether the opening of the housing of the sensor is restricted and whether the sensor is functioning normally.

11. The sensor of claim 10 wherein the controller is configured to determine whether the opening of the housing is restricted such that passage of gaseous fluids from the sensed environment through the opening is at least one of partially prohibited, substantially prohibited, or completely prohibited.

12. A method for operating the sensor according to any of the foregoing claims comprising:
taking a first reading of the environmental sensor to measure a first state of the at least one second ambient condition in the sensor environment;
operating the environment change catalyst;
taking a second reading of the environmental sensor to measure a second state of the at least one ambient second condition in the sensor environment;
polling the data table to find data representative of the second output of the environmental sensor; and
determining the operating condition of the sensor based on the data from the data table.

13. The method of claim 12 wherein determining the operating condition of the sensor comprises determining at least one of whether the opening of the housing of the sensor is restricted and whether the sensor is functioning normally.

14. The method of claim 12 wherein determining the operating condition of the sensor comprises determining whether the opening in the housing is restricted such that passage of gaseous fluids from the sensed environment through the opening is at least one of partially prohibited, substantially prohibited, or completely prohibited.

15. The method of any one of claims 12-14 further comprising comparing the first state to the second state and determining a change in state of the at least one second ambient condition of the sensor environment; and
wherein polling the data table comprises finding data representative of the change in state of the at least one second ambient condition of the sensor environment.
